# EUROPEAN PATENT SPECIFICATION

(11) **EP 0 860 166 B1**
(45) Date of publication and mention of the grant of the patent: **29.12.2004**
(21) Application number: 96932607.3
(22) Date of filing: 22.10.1996
(51) Int. Cl.: A61K 9/51

(54) **Nanoparticles based on hydrophilic polymers as pharmaceutical forms**
Nanopartikel auf der Basis hydrophiler Polymere als Arzneiformen
Nanoparticules à base de polymères hydrophiles comme formes pharmaceutiques

(30) Priority: 29.07.1996 ES 9601685
(43) Date of publication of application: 26.08.1998
(73) Proprietor: ADVANCELL - Advanced In Vitro Cell Tecnologies, 08028 Barcelona (ES)
(72) Inventor: ALONSO FERNANDEZ, Maria José, E-15706 Santiago de Compostela (ES); CALVO SALVE, Pilar, E-15706 Santiago de Compostela (ES); REMUNAN LOPES, Carmen, E-15706 Santiago de Compostela (ES); VILA JATO, José Luis, E-15706 Santiago de Compostela (ES)
(74) Representative: Davila Baz, Angel, C/O, Clarke, Modet & Co.
(86) International application number: PCT/ES1996/000186
(87) International publication number: WO 1998/004244

## Description

Application of nanoparticles based on hydrophilic polymers as pharmaceutical forms for the administration of active macromolecules.

The major constituent of these nanoparticles is a hydrophilic polymer: chitosan (which has a positive charge), besides the active ingredient, which may be an antigenic or therapeutic macromolecule (peptide, protein, antigen, oligonucleotide, RNA, DNA...). The nanoparticles may further comprise another hydrophilic polymer, polyoxyethylene (which has a non ionic character and tensoactives properties). The electrical charge of the colloidal particles can fluctuate between a positive value and a near to neutral value, depending on the relative ratio of the two polymers. The size of the particles is less than 1 micrometer.

Chitosan (a naturally occurring polymer), which shows an aminopolysaccharide structure and has a cationic character, is produced by a deacetilation process of the chitin (molecule that is obtained from crustacean shells). Chitosan is available in the market with various molecular weights and different degrees of deacetilation, in the form of chitosan base or chitosan salt (chlorhydrate or glutamate of chitosan).

Polyoxyethylene (PEO) is a synthetic non-ionic polymer. Polyoxyethylene and the block copolymers of ethylene oxide - propylene oxide (PEO-PPO) (poloxamers) are available in the market with different molecular weights and various ratios of ethylene to propylene groups. Their use in the preparation of injectable colloidal systems, specially the variety containing 80% ethylene oxide, is frequent because of their lack of toxicity.

The association efficiency of the active macromolecules to said particles is dependent on the system composition (ratio of both polymers) and on the physicochemical properties of the molecule which is intended to be associated.

The incorporation of the active macromolecules into the nanoparticles is achieved by a very simple and mild procedure which is particularly appropriate for preserving the delicate structure of the macromolecules. The incorporation is produced due to an interaction between the chitosan and the macromolecule which is intended to be associated. The formation of the nanoparticles is due to a process of simultaneous precipitation of chitosan and the active macromolecule, in the form of polymeric nanoaggregates, caused by the incorporation of an agent with a basic character, such as tripolyphosphate. In this method, the utilization of organic solvents, extreme pH conditions or auxiliary substances of toxic nature are not required.

The association of the active macromolecules with the nanoparticles occurs by a combined mechanism which may involve ionic and non-ionic interactions besides a physical entrapment process. The ionic interaction between chitosan and other polymers with opposite charge, has been the predominant mechanism involved in the formation of microcapsules by complex coacervation (T. Takahashi, K. Takayama, Y. Machida and N. Nagai, Chitosan-Alginate complex coacervate capsules: effects of calcium chloride, plasticizers and polyelectrolites on mechanical stability, Biotechnology Progress, 4, 76-81, 1988) and in the formation of complexes (M.M. Daly and D. Knoor, Characteristics of polyion complexes of chitosan with sodium alginate and sodium polyacrylate, Int. J. Pharm. 61, 35-41, 1990). However, the association of active macromolecules to chitosan or chitosan-PEO nanoparticles, according to an ionic interaction mechanism, has not yet been described. Besides, the incorporation of the bioactive macromolecules into the nanoparticles, is produced upon the incorporation into the medium of an agent inducing the crosslinking and precipitation of chitosan.

The current interest of hydrophilic nanoparticles is clearly illustrated by the growing amount of literature in this field. In this respect, it is worthwhile to mention several papers describing various methods of preparation of nanoparticles based on naturally occurring macromolecules (W. Lin, A.G.A. Coombes, M.C. Garnett, M.C. Davies, E. Stacht, S.S. Davis and L. Illum., Preparation of sterically stabilized human serum albumin nanospheres using a novel dextrano-MPEG crosslinking agent, Pharm. Res., 11, (1994)), (H.J. Watzke and C. Dieschbourg, Novel silica-biopolymer nanocomposites: the silica sol-gel process in biopolymer organogels , Adv. Colloid. Interface Sci., 50, 1-14, (1994)), (M. Rajaonarivony, C. Vauthier, G. Courrage, F. Puisiex and P. Couvreur, Development of a new drug carrier from alginate, J. Pharm. Sci., 82, 912-917, (1993)). However, the application of these nanoparticles for the association and controlled delivery of high molecular weight active ingredients such as peptides, proteins, antigens, oligonucleotides, etc., has not been described thus far. This could be due to the fact that most of the procedures described require the use of organic solvents, oils, high temperatures and crosslinking agents, circumstances that imply, in general, the destruction of this type of macromolecules. On the other hand, it has recently also been proposed the use of hydrophilic synthetic nanoparticles made of copolymers of lactic acid and PEO, for the association and controlled delivery of peptides and proteins (P. Quellec, R. Gref P. Calvo, M.J. Alonso and E. Dellacherie, Encapsulation of a model protein and a hydrophobic drug into long-circulating biodegradable nanospheres, Proceed. Intern. Symp. Control. Rel. Bioact. Mater., 23, (1996), CSR, Inc). Once again, however, the main limitation of these nanoparticles is the necessity of using organic solvents of toxic nature in the preparation thereof.

Despite the efforts that the researchers have dedicated to the formulation of active macromolecules, no reference has been found in the bibliography and patents revision dealing with the application of chitosan or chitosan-PEO or chitosan-PEO-PPO nanoparticles for the association and controlled delivery of macromolecules with therapeutic or antigenic interest.

US-A-5,346,703 discloses a gel composition that consists of a mixture of an ionic saccharide, a polyoxyalkylene block copolymer and, optionally but not necessarily, a counter-ion capable of crosslinking the ionic polysaccharide. The disclosed aqueous gels are characterized in that they undergo a sol-gel transition upon changes in temperature. More specifically, this aqueous solution has, upon preparation, a low viscosity and following its administration *in vivo* it will be converted into a viscous gel. This well known sol-gel transition is temperature dependent.

WO-A-96/05810 discloses a drug delivery composition comprising particles of chitosan or a chitosan derivative or salt, the particles being either solidified or partially cross-linked. Further, these particles are said to be microspheres, with a size in the range of 1 to 200 µm, more preferably 1 to 100 µm. It discloses that the "solidifies particles" may be prepared by emulsification of an aqueous solution of chitosan in an oil. Then, sodium hydroxide is added in order to precipitate the particles. Further, "partially cross-linked particles" are disclosed. These partially cross-linked particles may be prepared by emulsification or spray drying techniques, followed by cross-linking. Even though sodium tripolyphosphate is mentioned in WO-A-96/05810 as a possible crosslinking agent, there is not one single example of the use of this agent. All examples refer to the use of aldehydes as covalent crosslinking agents.

WO-A-96/20698 discloses nanoparticles, which are prepared by subjecting a polymeric material to an organic solvent emulsification process. Thus, a solution in an organic solvent is always required. Although chitosan is mentioned in WO-A-96/20698, there are no working examples of nanoparticles prepared using chitosan.

In a first aspect, the present invention relates to a pharmaceutical composition, comprising nanoparticles, according to the features of claim 1.
In a second aspect, the invention relates to a process for the preparation of the nanoparticles, according to the features of claim 12.
Further, claims 2 to 11 relate to preferred embodiments of the composition of claim 1.
Claims 12 to 15 further relate to preferred embodiments of the process of claim 12.

The present invention solves the problems raised in the previous developments, achieving the formation of a new pharmaceutical composition based upon the association of active macromolecules to nanoparticles with an extremely hydrophilic character. The main component of the nanoparticles carrying active macromolecules is a hydrophilic polymer, chitosan or any derivatives thereof. The nanoparticles may further comprise another hydrophilic polymer, the PEO or any derivatives thereof. The presence of polyoxyethylene is not necessary for the obtaining of the particles, but it allows to modify the physicochemical properties of the same (particles size and zeta potential), to modulate the delivery of the macromolecule incorporated, and to improve the biocompatibility of the chitosan nanoparticles. The ratio of both polymers can be very variable; the ratio of one of them may be 50 times higher than the other one. The ratio in which the active ingredient (peptide or protein) associates can reach values of 100% (association efficiency).

The colloidal systems or nanoparticles which are proposed for the association and controlled delivery of active macromolecules show numerous advantages over other types of nanoparticles previously described in the literature, not only from the point of view of their preparation, but also from the point of view of their interest as new pharmaceuticat forms, administrable by all ways of administration actually conceived. The most important benefits of these new formulations include: (1) the procedure for the incorporation of the active macromolecule into the nanoparticles is simple and does not require the use of ingredients which are toxic for the organism, such as organic solvents and oils; (2) their physicochemical properties, more specifically, their size, hydrophilia and surface charge, can be modulated by simply adjusting the ratio of chitosan-PEO; (3) these nanoparticles show an extraordinary capacity for the association of therapeutic and antigenic macromolecules, and (4) they deliver the associated active macromolecule at a controlled speed.

Within the routes for which these systems are interesting, on the one hand, those stand out wherein the contact of the nanoparticles with an epithelial surface such as the oral, transdermal, ocular, nasal and vaginal routes is involve, and, on the other hand those which involve an injection. In the first case the contact of these colloidal particles with the epithelium, may be favored by providing the nanoparticles with a high positive charge, what would favor their interaction with the cited mucosal surfaces which are negatively charged. In the second case, especially for the intravenous administration, these systems offer the possibility of modulating the distribution in vivo of the drugs or active molecules associated with them.

Therefore, in the invention we provide new compositions of pharmaceutical interest for the administration of active macromolecules. These systems, constituted by a nanoparticles suspension, can be presented in a liquid form of variable viscosity. The nanoparticles are constituted based upon chitosan (in the form of chitosan base or chitosan salt) and, optionally, poloxamer polymers, and they can contain a variable amount of an active macromolecule of therapeutic or antigenic character. Although the incorporation of active macromolecules into the nanoparticles only formed based upon chitosan is possible, the inclusion of the poloxamer polymer in the system allows to modulate the characteristics of the nanoparticles in terms of particle size, zeta potential and hydrophilicity (as may be observed in Table 1).

The most outstanding aspect of the present invention is the one that refers to the application of the chitosan nanoparticles as administration forms of active ingredients of high molecular weight and of hydrophilic character. This aspect is of extraordinary interest if we keep in mind that most of the nanoparticles systems patented at the present time, only allow the encapsulation of medicines and other ingredients of lipophilic character.

By active ingredient, the ingredient for which the formulation is designed is meant, that is, the ingredient that has to fulfill a particular function following its administration to an alive organism. The function may be to combat, palliate or prevent a disease (vaccines, vitamins...), to improve the physical and esthetical appearance (moisturizing of the skin, preventing or facilitating the loss of the hair...) and similar.

The pharmaceutical systems described are characterized in that they have a size smaller than 1 µm, reason what for they are called nanoparticles, and in that they show a great capacity for the association of macromolecules. It has been observed that the size of the particles is highly dependent on the chitosan concentration in the aqueous medium in which the nanoparticles are formed; in fact, for too low chitosan concentrations (lower than 0.01% in aqueous phase) or too high chitosan concentrations (higher than 0.5% in aqueous phase), only a solution or big particles of high size (larger than 1 mm) respectively is obtained. In addition, the size of the nanoparticles is highly influenced by the poloxamer concentration, with which the chitosan interacts. For example, as shown in Table 1, when the ratio of chitosan/poloxamer increases from 1/0 to 1/50, a considerable increment of the particle (from 275nm to 685nm) and a reduction of the zeta potential value are produced.

Using albumin as a model of therapeutic macromolecule, a high association efficiency of said molecule to the poloxamer-chitosan nanoparticles was shown, being observed that said efficiency is dependent on the albumin concentration and on the presence of poloxamer in the aqueous medium in which the nanoparticles are formed (Table 2). Table 2 also shows that the incorporation of albumin into the cited nanoparticles does not lead to important modifications neither in the particle size nor in the zeta potential. On the other hand, it was observed that the stage at which the albumin was incorporated into the nanoparticles has a remarkable effect on its efficiency, the maximum association efficiency being observed when the protein is incorporated into the tripolyphosphate (TTP) crosslinking agent solution and the minimum efficiency when the albumin is incorporated into the previously formed nanopartcles(table 3). Likewise, it may be outstood that the pH of the medium in which the association of the protein to the nanoparticles occurs, plays an essential role in the association efficiency of the albumin to the nanoparticles (table 4)

An important aspect of the chitosan nanoparticles application as pharmaceutical systems, is their capacity to deliver the active macromolecule associated into them for extended periods of time. Furthermore, said nanoparticles deliver the associated macromolecule to a rate that can be modulated based upon the presence of poloxamer and by the amount of the active macromolecule associated to them.

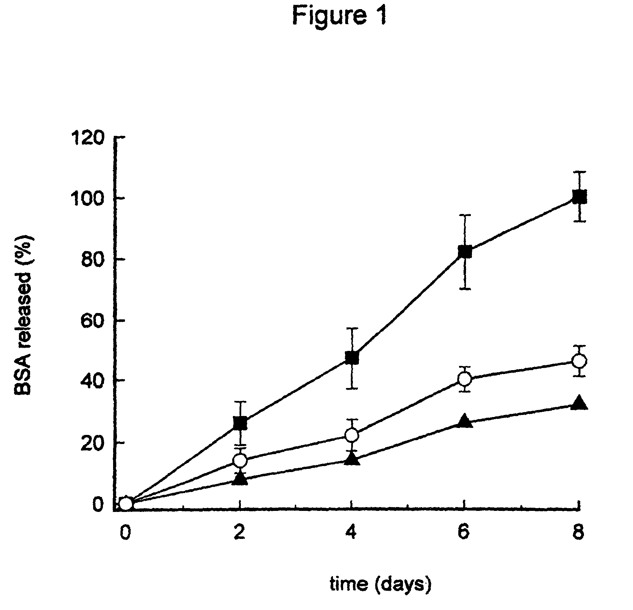

Figure 1 displays releasing of albumin from chitosan nanoparticles with different concentrations of poloxamer: (■) chitosan/poloxamer 1/0; (○) chitosan/poloxamer 1/5; (▲) chitosan/poloxamer 1/25. Representing in the abcissas the time (days) and in ordinates the amount of albumin released, expressed in percentage (%).

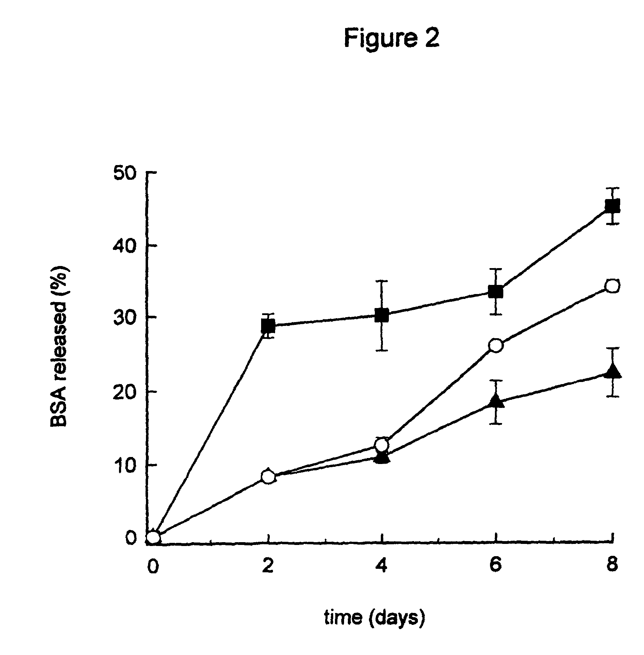

Figure 2 displays the releasing of albumin from chitosan nanoparticles containing different albumin loading : (Δ) 41% albumin/chitosan; (●) 25% albumin/chitosan ; (□) albumin/chitosan 20%. Representing in the abcissas the time (days) and in ordinates the amount of albumin released, expressed in percentage (%).

On the other hand, the application of the chitosan nanoparticles as systems carrying antigenic macromolecules, has been shown, using the tetanus and diphtheria toxoids. Results of the association efficiency of the two antigens into the nanoparticles are presented in Table 5. Also, studies of release "in vitro" of the antitetanic vaccine have revealed the release of the vaccine from the nanoparticles in their active form.

To sum up, the present invention covers a new pharmaceutical composition which can be used for the administration of active macromolecules by different routes including topical, oral, nasal, pulmonary, vaginal, ocular, subcutaneous, intramuscular and intravenous.

### Example 1

Association of albumin (model protein) to the chitosan nanoparticles.

A nanoparticles suspension of the following composition in % (w/w) was prepared:

| | |
|---|---|
| Chitosan | 0.14 % |
| Tripolyphosphate | 0.02 % |
| Albumin (BSA) | 0.014% |
| Water | up to 100% |

The preparation was carried out as follows:

25ml of an acid aqueous solution (0.05 M acetic acid) of 0.2% (w/v) chitosan were prepared and the solution was adjusted to pH 5. Afterwards, 5 mg of albumin (BSA, bovine serum albumin) were incorporated and then 10 ml of tripolyphosphate solution (0.1%) were added under magnetic stirring at 100 rpm. The suspension was maintained under continuous stirring during 30 minutes.

Once the nanoparticles were obtained, their particle size, Z potential and association efficiency of the albumin expressed as the quantity of albumin associated to the nanoparticles per quantity used in the elaboration of the same were determined. The values obtained for the mentioned parameters are respectively 402 nm, 46 mV and 100%.

### Example 2

Association of the albumin to the nanoparticles of chitosan/PEO-PPO at a 1/5 (w/w) ratio.

A similar formulation as described in example 1 was prepared but containing PEO-PPO. The procedure was similar to the one described previously having incorporated the PEO-PPO to the chitosan solution at pH=4 previously to the incorporation of the albumin.

| | |
|---|---|
| Chitosan | 0.14 % |
| PEO-PPO | 0.70 % |
| Albumin | 0.014% |
| Tripolyphosphate | 0.02 % |
| Water | up to 100% |

The results of particle size, zeta potential and albumin association efficiency were 519 nm, 43.6 mV and 78.2%.

### Example 3

Association of albumin to nanoparticles of chitosan/PEO-PPO at a ratio of 1/25 (w/w).

A similar formulation was prepared as described in example 2, but containing an amount 5 times higher of PEO-PPO. The procedure was identical to the one described previously.

| | |
|---|---|
| Chitosan | 0.14 % |
| PEO-PPO | 3.50 % |
| Albumin | 0.014% |
| Tripolyphosphate | 0.02 % |
| Water | up to 100% |

The results of particle size, zeta potential and albumin association efficiency were 741 nm, 33.9 mV and 45.9 %, respectively.

### Example 4

Association of the tetanus toxoid to chitosan nanoparticles:

A formulation, similar to the one described in example 1, but containing tetanus toxoid in the indicated proportion, was prepared. The procedure was identical to the one described previously.

| | |
|---|---|
| Chitosan | 0.14 % |
| TetanusToxoid | 0.014% |
| Tripolyphosphate | 0.02 % |
| Water | up to 100% |

The results of particle size, zeta potential and tetanus toxoid association efficiency were 245 nm, 35 mV and 53 %, respectively.

### Example 5

Association of the diphtheria toxoid to chitosan nanoparticles:

A similar formulation as described in example 1 was prepared but containing diphtheria toxoid in the indicated proportion. The produce was identical to the one described previously.

| | |
|---|---|
| Chitosan | 0.14 % |
| Diphtheria Toxoid | 0.007% |
| Tripolyphosphate | 0.02 % |
| Water | up to 100% |

The results of particle size, the zeta potential and the toxoid association efficiency were 245 nm, 35.7 mV and 55 %, respectively.

**Table 1:**

| Values of particle size and zeta potential obtained for intermediate chitosan/PEO-PPO ratios. | | |
|---|---|---|
| Chitosan/ PEO-PPO (w/w) | Size (nm)* | Zeta potential (mV)^{#} |
| 1/0 | 275 | 43.96 |
| 1/2.5 | 283 | 41.24 |
| 1/5 | 300 | 40.54 |
| 1/25 | 430 | 27.99 |
| 1/50 | 685 | 17.89 |

| | | |
|---|---|---|
| * Determined by Photon Correlation Spectroscopy | | |
| # Determined by Laser Beam Scattering | | |

**Table 2 :**

| Association efficiency of albumin to the nanoparticles with the composition previously described. | | | |
|---|---|---|---|
| Chitosan/Albumin (w/w) | Size (nm)* | Zeta potential (mV)^{#} | associated (%) |
| 10/1 | 405 +/- 24 | 45 +/- 1 | 80 |
| 4/1 | 359 +/- 35 | 45 +/- 1 | 43 |
| 2/1 | 375 +/- 26 | 45 +/- 1 | 26 |
| 1/1 | 368 +/- 72 | 46 +/- 2 | 21 |

| | | | |
|---|---|---|---|
| * Determined by Photon Correlation Spectroscopy | | | |
| # Determined by Laser Beam Scattering | | | |

**Table 3:**

| Association efficiency of albumin (BSA) to the chitosan nanoparticles as a function of the stage at which albmin was incorporated. | | | |
|---|---|---|---|
| Chitosan/BSA | | % BSA associated | |
| | Nanoparticles | BSA + chitosan | BSA + TPP |
| 10/1 | | 45 ± 1 | 100 |
| 2/1 | 10.8 | 45 ± 1 | 45.2 |
| 1/1 | | 45 ± 1 | 41.8 |

**Table 4:**

| Association efficiency of albumin to chitosan nanoparticles as a function of the pH of the chitosan solution. | | | |
|---|---|---|---|
| Chitosan/ Albumin (w/w) | % associated | | |
| | pH 3 | pH 4 | pH 5 |
| 10/1 | 66.8 | 80.4 | 91.7 |
| 2/1 | 25.7 | 26.8 | 39.1 |
| 1/1 | 19.4 | 21.6 | 35.5 |

The association efficiency results of the tetanus toxoid and diphtheria toxoid to the chitosan nanoparticles are shown in table 5. The influence of the pH of the chitosan solution and the tetanus toxoid concentration on the association degree of the same to the chitosan nanoparticles, is indicated in said table.

**Table 5:**

| Toxoid | Chitosan/Toxoid (w/w) | pH | % Associated |
|---|---|---|---|
| Tetanus | 1/0.06 | 4 | 4.27 |
| Tetanus | 1/0.06 | 4 | 15.9 |
| Tetanus | 1/0.12 | 5 | 53.3 |
| Diphtheria | 1/0.12 | 5 | 55.1 |

## Claims

1. Pharmaceutical compositions for the administration of high molecular weight active ingredients comprising nanoparticles having a size less than 1 micrometer made of hydrophilic polymers, **characterised in that** the main ingredients are:
a) chitosan;
b) a crosslinking agent with a basic character, and
c) a high molecular weight active ingredient,
wherein the chitosan is cross-linked and precipitated with the crosslinking agent with a basic character.

2. Pharmaceutical composition according to claim 1, **characterised in that** the nanoparticles further comprise a polyoxyethylene or derivative thereof.

3. Pharmaceutical composition, according to claim 1, **characterised in that** the nanoparticles have a positive electrical charge or zeta potential and a capacity to incorporate active ingredients and proteins.

4. Pharmaceutical composition, according to claim 2, **characterised in that** the polyoxyethylene derivative is selected from the group consisting of the polyoxyethylene and the copolymers of the ethylene oxide and propylene oxide.

5. Pharmaceutical composition, according to any one of claims 1 to 4, **characterised in that** the high molecular weight active ingredient is selected form the group of macromolecules consisting of peptides, proteins, polysaccharides and nucleotides, having a therapeutic or antigenic activity.

6. Pharmaceutical composition, according to claim 5, **characterised in that** the active ingredient is tetanus toxoid.

7. Pharmaceutical composition, according to claim 5, **characterised in that** the active ingredient is diphteria toxoid.

8. Pharmaceutical composition according to any of claims 1 to 7, **characterised in that** the crosslinking agent is tripolyphosphate.

9. Pharmaceutical composition, according to claim 4, **characterised in that** the percentage of polyoxyethylene with respect to the total weight of the ingredients is comprised between 0 % and 60 % in weight.

10. Pharmaceutical composition, according to claim 5, **characterised in that** the quantity of active ingredient with respect to the total weight of the ingredients, expressed in percentage, is comprised between 0 % and 66 % in weight.

11. Pharmaceutical composition, according to the claims 1 to 10, **characterised in that** they are prepared in an appropriate form for their application by oral, nasal, vaginal, pulmonary and topical routes, the particles having a high positive charge that facilitates their interaction with epithelial or mucosal surfaces.

12. A process for the preparation of nanoparticles having a size less than 1 micrometer and comprising chitosan and a high molecular weight active ingredient, comprising forming said nanoparticles in an aqueous medium by cross-linkage and precipitation of chitosan with a cross-linking agent of a basic character and simultaneously or subsequently contacting said nanoparticles with said active ingredient.

13. A process according to claim 12 wherein the nanoparticles are formed in the presence of a polyoxyethylene or derivative thereof.

14. A process according to claim 13 wherein the nanoparticles are formed in the presence of polyoxyethylene or a copolymer of ethylene oxide and propylene oxide.

15. A process according to any one of claims 12 to 14 wherein the cross-linking agent is tripolyphosphate.

## Patentansprüche

1. Pharmazeutische Zusammensetzungen zur Verabreichung von hochmolekularen aktiven Bestandteilen, die Nanopartikel mit einer Größe von weniger als 1 Mikrometer umfassen, die aus hydrophilen Polymeren hergestellt wurden, **dadurch gekennzeichnet, dass** die Hauptbestandteile
a) Chitosan;
b) ein Vernetzungsmittel mit basischem Charakter; und
c) ein hochmolekularer aktiver Bestandteil sind,
wobei das Chitosan mit dem Vernetzungsmittel mit basischem Charakter vernetzt und präzipitiert ist.

2. Pharmazeutische Zusammensetzung gemäß Anspruch 1, **dadurch gekennzeichnet, dass** die Nanopartikel weiter ein Polyoxyethylen oder ein Derivat davon umfassen.

3. Pharmazeutische Zusammensetzung gemäß Anspruch 1, **dadurch gekennzeichnet, dass** die Nanopartikel eine positive elektrische Ladung oder Zeta-Potential und ein Aufnahmevermögen zum Einlagern aktiver Bestandteile und Proteine aufweisen.

4. Pharmazeutische Zusammensetzung gemäß Anspruch 2, **dadurch gekennzeichnet, dass** das Polyoxyethylenderivat aus der Gruppe, die aus dem Polyoxyethylen und den Copolymeren des Ethylenoxids und Propylenoxids besteht, ausgewählt wird.

5. Pharmazeutische Zusammensetzung gemäß einem der Ansprüche 1 bis 4, **dadurch gekennzeichnet, dass** der hochmolekulare aktive Bestandteil aus der Gruppe der Makromoleküle, die aus Peptiden, Proteinen, Polysacchariden und Nukleotiden mit einer therapeutischen oder antigenischen Aktivität besteht, ausgewählt wird.

6. Pharmazeutische Zusammensetzung gemäß Anspruch 5, **dadurch gekennzeichnet, dass** der aktive Bestandteil ein Tetanustoxoid ist.

7. Pharmazeutische Zusammensetzung gemäß Anspruch 5, **dadurch gekennzeichnet, dass** der aktive Bestandteil ein Diphterietoxoid ist.

8. Pharmazeutische Zusammensetzung gemäß einem der Ansprüche 1 bis 7, **dadurch gekennzeichnet, dass** das Vernetzungsmittel ein Tripolyphosphat ist.

9. Pharmazeutische Zusammensetzung gemäß Anspruch 4, **dadurch gekennzeichnet, dass** der Prozentsatz an Polyoxyethylen in Bezug auf das Gesamtgewicht der Bestandteile zwischen 0% und 60% des Gewichts umfasst.

10. Pharmazeutische Zusammensetzung gemäß Anspruch 5, **dadurch gekennzeichnet, dass** die Menge des aktiven Bestandteils in Bezug auf das Gesamtgewicht der Bestandteile, das in Prozent ausgedrückt wird, zwischen 0% und 66% des Gewichts umfasst.

11. Pharmazeutische Zusammensetzung gemäß den Ansprüchen 1 bis 10, **dadurch gekennzeichnet, dass** sie in einer geeigneten Form für ihre Anwendung über orale, nasale, vaginale, pulmonale und topische wege zubereitet worden sind, wobei die Partikel eine hohe positive Ladung aufweisen, die ihre Wechselwirkung mit epithelialen oder mucosalen Oberflächen erleichtert.

12. Ein Verfahren zum Herstellen von Nanopartikeln, die eine Größe von weniger als 1 Mikrometer aufweisen und die Chitosan und einen hochmolekularen Bestandteil umfassen, welches umfasst, die Nanopartikel in einem wässrigen Medium durch Vernetzung und Präzipitierung von Chitosan mit einem Vernetzungsmittel mit basischem Charakter zu erzeugen und die Nanopartikel gleichzeitig oder aufeinanderfolgend mit dem aktiven Bestandteil in Kontakt zu bringen.

13. Ein Verfahren gemäß Anspruch 12, wobei die Nanopartikel in Anwesenheit eines Polyoxyethylens oder eines Derivates davon gebildet werden.

14. Ein Verfahren gemäß Anspruch 13, wobei die Nanopartikel in Anwesenheit von Polyoxyethylen oder einem Copolymer des Ethylenoxids und Propylenoxids gebildet werden.

15. Ein Verfahren gemäß einem der Ansprüche 12 bis 14, wobei das Vernetzungsmittel ein Tripolyphosphat ist.

## Revendications

1. Compositions pharmaceutiques pour l'administration d'ingrédients actifs à haut poids moléculaire, comprenant des nanoparticules ayant une grosseur inférieure à 1 micromètre réalisés en polymères hydrophiles, **caractérisées par le fait que** les ingrédients principaux sont :
a) chitosane ;
b) un agent de réticulation de nature basique ; et
c) un ingrédient actif à haut poids moléculaire,
dans lesquelles le chitosane est réticulé et précipité avec l'agent de réticulation de nature basique.

2. Composition pharmaceutique selon la revendication 1, **caractérisée par le fait que** les nanoparticules comprennent, par ailleurs, un polyoxyéthylène ou un dérivé de celui-ci.

3. Composition pharmaceutique selon la revendication 1, **caractérisée par le fait que** les nanoparticules ont une charge électrique positive ou potentiel électrocinétique et une capacité pour incorporer des ingrédients actifs et des protéines.

4. Composition pharmaceutique selon la revendication 2, **caractérisée par le fait que** le dérivé de polyoxyéthylène est choisi parmi le groupe composé de polyoxyéthylène et des copolymères de l'oxyde d'éthylène et de l'oxyde de propylène.

5. Composition pharmaceutique selon l'une quelconque des revendications 1 à 4, **caractérisée par le fait que** l'ingrédient actif à haut poids moléculaire est choisi parmi le groupe de macromolécules composées de peptides, de protéines, de polysaccharides et de nucléotides ayant une activité thérapeutique ou antigène.

6. Composition pharmaceutique selon la revendication 5, **caractérisée par le fait que** l'ingrédient actif est le précipité tétanique.

7. Composition pharmaceutique selon la revendication 5, **caractérisée par le fait que** l'ingrédient actif est le précipité diphtérique.

8. Composition pharmaceutique selon l'une quelconque des revendications 1 à 7, **caractérisée par le fait que** l'agent de réticulation est le tripolyphosphate.

9. Composition pharmaceutique selon la revendication 4, **caractérisée par le fait que** le pourcentage de polyoxyéthylène par rapport au poids total des ingrédients est compris entre 0 % et 60 % en poids.

10. Composition pharmaceutique selon la revendication 5, **caractérisée par le fait que** la quantité d'ingrédient actif par rapport au poids total des ingrédients, exprimée en pourcentage, est compris entre 0 % et 66 % en poids.

11. Composition pharmaceutique selon les revendications 1 à 10, **caractérisée par le fait qu'**elle est préparée sous une forme appropriée pour leur application par voies orale, nasale, vaginale, pulmonaire et topique, les particules ayant une haute charge positive qui facilite leur interaction avec les surfaces épithéliales ou muqueuses.

12. Procédé pour la préparation de nanoparticules ayant une grosseur inférieure à 1 micromètre et comprenant du chitosane et un ingrédient actif à haut poids moléculaire, comprenant la formation desdites nanoparticules dans un milieu aqueux par réticulation et précipitation de chitosane avec un agent de réticulation de nature basique et la mise en contact, simultanément ou ultérieurement, desdites nanoparticules avec ledit ingrédient actif.

13. Procédé selon la revendication 12, dans lequel les nanoparticules sont formées en présence d'un polyoxyéthylène ou un dérivé de celui-ci.

14. Procédé selon la revendication 12, dans lequel les nanoparticules sont formées en présence de polyoxyéthylène ou d'un copolymère d'oxyde d'éthylène et d'oxyde de propylène.

15. Procédé selon l'une quelconque des revendications 12 à 14, dans lequel l'agent de réticulation est le tripolyphosphate.
